# EUROPEAN PATENT APPLICATION

(11) **EP 3 815 636 A1**
(43) Date of publication of application: **05.05.2021**
(21) Application number: 18924220.9
(22) Date of filing: 26.12.2018
(51) Int. Cl.: A61B 17/34

(54) **ULTRASOUND-GUIDED ASSISTANCE DEVICE AND SYSTEM FOR NEEDLE**

(30) Priority: 27.06.2018 CN 201810680946
(71) Applicant: CHISON Medical Technologies Co., Ltd., Wuxi, Jiangsu 214142 (CN)
(72) Inventor: MO, Ruoli, Wuxi, Jiangsu 214142 (CN); HUANG, Mingjin, Wuxi, Jiangsu 214142 (CN); ZHAO, Mingchang, Wuxi, Jiangsu 214142 (CN)
(74) Representative: Finnegan Europe LLP
(86) International application number: PCT/CN2018/124034
(87) International publication number: WO 2020/000963

(57) **Abstract**

The present invention provides an ultrasonic guidance auxiliary device for a needle, including: a probe, used for emitting and receiving an ultrasonic signal for a tissue to be punctured-for-injection; a first mechanical arm, used for moving the probe to the tissue to be punctured-for-injection; a second mechanical arm, used for fixing a puncture needle, moving the needle to be close to the tissue to be punctured-for-injection, and then causing the needle to puncture the tissue to be punctured-for-injection; and a main unit, used for moving, according to controlling of the first mechanical arm, the probe to the tissue to be punctured-for-injection, wherein the probe emits and receives the ultrasonic signal. The main unit synthesizes an ultrasonic image through the ultrasonic signal transmitted by the probe, and the main unit at least calculates a puncture distance and a puncture angle between the needle and the tissue to be punctured-for-injection through the ultrasonic image. The main unit controls, according to the puncture distance and the puncture angle, the second mechanical arm to move the needle to the corresponding puncture distance and puncture angle to perform puncturing. The ultrasonic guidance auxiliary device is used for guiding accurate puncturing of the needle.

## Description

### Field

The present invention relates to a medical guidance auxiliary device and system, in particular, a medical ultrasonic guidance auxiliary device and system.

### Background

At present, the application of ultrasonic equipment in clinical diagnosis and treatment has been very popular, which has made a great contribution to doctors to accurately understand patients' conditions, formulate medical plans, and assist treatment.

At present, ultrasound has been widely used for puncture guidance, but a probe is still manually implemented to perform ultrasonic probe scanning on the surface of a human tissue to be tested, then ultrasonic imaging is manually performed to determine a depth of blood vessels and other tissues, and then manual puncture is performed. This requires three manual determination operations, such as how to perform fast ultrasonic probe scanning on an effective region, how to determine the depths of other tissues such as blood vessels on an ultrasonic image, and how to select a suitable puncture position and a suitable puncture angle for puncturing.

Intravenous injection is different from the current routine departmental puncture. At present, ultrasound or infrared is used to guide the puncture. A puncture needle is often an independent needle tube. Routine intravenous injections (such as infusion) have not yet been guided by ultrasound in the market. Intravenous injections are the most routine work in hospitals or clinics. Doctors or nurses need to perform a large number of intravenous injections every day. Because of human experience, it is inevitable that intravenous injection failures often occur due to doctors' or nurses' negligence or misjudgment. For example, an intravenous injection needle punctures a blood vessel, or a very thin blood vessel is selected, or an inappropriate intravenous injection angle affects the stability of intravenous injection liquor entering any blood vessel or tissue.

### Summary

The present invention is directed to overcome the shortcomings in the prior art, and provides an ultrasonic guidance auxiliary device and system for a needle, which are used for guiding accurate puncturing of the needle. The technical solution adopted by the present invention is as follows:
An ultrasonic guidance auxiliary device for a needle includes:
a probe, used for emitting and receiving an ultrasonic signal for a tissue to be punctured-for-inj ection;
a first mechanical arm, used for moving the probe to the tissue to be punctured-for-inj ection;
a second mechanical arm, used for fixing a puncture needle, moving the needle to be close to the tissue to be punctured-for-injection, and then causing the needle to puncture the tissue to be punctured-for-injection; and
a main unit, used for moving, according to controlling of the first mechanical arm, the probe to the tissue to be punctured-for-injection, wherein the probe emits and receives the ultrasonic signal.

The main unit synthesizes an ultrasonic image through the ultrasonic signal transmitted by the probe, and the main unit at least calculates a puncture distance and a puncture angle between the needle and the tissue to be punctured-for-injection through the ultrasonic image.

The main unit controls, according to the puncture distance and the puncture angle, the second mechanical arm to move the needle to the corresponding puncture distance and puncture angle to perform puncturing.

Further, the main unit acquires, according to the ultrasonic signal transmitted by the probe, a position of the needle in the tissue to be punctured-for-injection, and controls the second mechanical arm to maintain the sharp end of the needle in a target region of the tissue to be punctured-for-injection.

Much further, the main unit controls the first mechanical arm and the second mechanical arm to cooperatively move to always maintain the sharp end of the needle to a position that can be detected by the probe, and controls the second mechanical arm to maintain the sharp end of the needle in the target region of the tissue to be punctured-for-injection.

Further, the tissue to be punctured-for-injection includes one or more of an artery, a vein, a nerve, and an organ.

Much further, the main unit acquires a minimum sectional area of the artery or the vein according to an ultrasonic image of the artery or the vein, and the main unit controls the probe by means of the first mechanical arm to be orthogonal to the artery or the vein.

Further, the main unit calculates, according to the ultrasonic image, a three-dimensional coordinate of a center point in the tissue to be punctured-for-injection, and the main unit calculates, according to the three-dimensional coordinate of the center point, the puncture distance and the puncture angle between the needle and the tissue to be punctured-for-inj ection.

Further, the first mechanical arm includes a rotating articulated arm, and the rotating articulated arm controls the probe to rotate to enable the main unit to obtain an ultrasonic image of the tissue to be punctured-for-injection.

Further, the ultrasonic guidance device for the needle includes an infrared radiation light source; the infrared radiation light source is used for performing infrared radiation on the tissue to be punctured-for-injection; and the main unit further includes an infrared image collection unit used for controlling a camera to acquire an infrared image of the tissue to be punctured-for-inj ection.

Further, the second mechanical arm and the first mechanical arm are separately mounted on corresponding bases or mounted on a same base; or,
the second mechanical arm is fixedly mounted on the first mechanical arm.

After controlling the second mechanical arm to cause the needle to puncture a human tissue, the main unit controls the second mechanical arm to cause the needle to continue to puncture the human tissue.

The ultrasonic guidance auxiliary device includes a display, used for displaying the ultrasonic image obtained by scanning of the probe, and a dynamic puncture ultrasonic image after the needle punctures the human tissue.

The present invention provides an ultrasonic guidance auxiliary system for a needle, including:
a probe, used for emitting and receiving an ultrasonic signal for a tissue to be punctured-for-inj ection;
a first mechanical arm, used for moving the probe to the tissue to be punctured-for-inj ection;
a second mechanical arm, used for fixing a puncture needle, and moving the needle to the tissue to be punctured-for-injection, and then causing the needle to puncture the tissue to be punctured-for-injection; and
a main unit, wherein the main unit includes a first mechanical arm control unit, a second mechanical arm control unit, a probe control unit, an image processing unit, and a displacement calculation unit; the first mechanical arm control unit controls the first mechanical arm to move the probe to the tissue to be punctured-for-injection, and the probe control unit controls the probe to emit and receive the ultrasonic signal.

The image processing unit synthesizes an ultrasonic image through the ultrasonic signal transmitted by the probe;
The displacement calculation unit at least calculates a puncture distance and a puncture angle between the needle and the tissue to be punctured-for-injection through the ultrasonic image.

The second mechanical arm control unit controls, according to the puncture distance and the puncture angle, the second mechanical arm to move the needle to the corresponding puncture distance and puncture angle to perform puncturing.

Further, the displacement calculation unit acquires, according to the ultrasonic image, a position of the needle in the tissue to be punctured-for-injection, and the second mechanical arm control unit controls the second mechanical arm to maintain the sharp end of the needle in a target region of the tissue to be punctured-for-injection.

Much further, the displacement calculation unit acquires, according to the ultrasonic image, a position of the needle in the tissue to be punctured-for-injection; the first mechanical arm controlled by the first mechanical arm control unit and the second mechanical arm controlled by the second mechanical arm control unit perform cooperative movement to always maintain the needle at a position that can be detected by the probe, and the sharp end of the needle is maintained in the target region of the tissue to be punctured-for-injection.

The tissue to be punctured-for-injection includes one or more of an artery, a vein, a nerve, and an organ.

The displacement calculation unit acquires a minimum sectional area of the artery or the vein according to an ultrasonic image of the artery or the vein, and the displacement calculation unit controls the probe by means of the first mechanical arm to be orthogonal to the artery or the vein.

The displacement calculation unit calculates, according to the ultrasonic image, a three-dimensional coordinate of a center point in the tissue to be punctured-for-injection, and the displacement calculation unit calculates, according to the three-dimensional coordinate of the center point, the puncture distance and the puncture angle between the needle and the tissue to be punctured-for-injection.

Further, the ultrasonic guidance auxiliary system for the needle further includes an infrared radiation light source. The main unit further includes an infrared light source processing unit used for controlling the infrared radiation light source to radiate infrared ray. The ultrasonic guidance auxiliary system further includes a camera. The main unit further includes an infrared image collection unit used for controlling the camera to acquire an infrared image of the tissue to be punctured-for-injection.

The image processing unit includes: an image fusion processing unit, an ultrasonic image processing unit, and an infrared image processing unit. The image fusion processing unit fuses the ultrasonic image obtained by the ultrasonic image processing unit with the infrared image obtained by the infrared image processing unit to generate a new image.

The ultrasonic guidance auxiliary system for the needle includes a display used for displaying the ultrasonic image obtained by scanning of the probe and a dynamic puncture ultrasonic image after the needle punctures the human tissue, and displaying the infrared image.

The present invention has the advantages that: the present invention performs automatic scanning through the ultrasonic probe connected with the first mechanical arm connected with the main unit; the main unit processes the ultrasonic image according to a signal obtained by the probe; the main unit automatically determines, according to the processed ultrasonic image, parameters, such as the depth and the diameter of the blood vessel; the main unit controls the needle connected with the second mechanical arm to be automatically inserted into a target blood vessel or tissue; as the second mechanical arm controls an intravenous injection needle to be continuously inserted into the blood vessel or issue, the main unit controls the probe on the first mechanical arm to cooperate with a motion track of the needle on the second mechanical arm to move, so as to ensure that the needle on the second mechanical arm to be always inserted into a position near the center of the blood vessel.

By means of the above technical solution, the present invention solves a problem that how to select a blood vessel with a proper thickness and shape, and ensures the comfort at the later stage of intravenous injection.

### Brief Description of the Drawings

Fig. 1 is a schematic structural diagram of an exemplary ultrasonic guidance auxiliary device of the present invention;
Fig. 2 is a schematic structural diagram of an ultrasonic guidance auxiliary device including an infrared radiation light source according to the present invention;
Fig. 3 is another exemplary schematic structural diagram of working states of a first mechanical arm and a second mechanical arm of the present invention;
Fig. 4 is another exemplary structural sectional diagram of working states of a first mechanical arm and a second mechanical arm of the present invention;
Fig. 5 is a schematic diagram after a needle punctures a blood vessel of the present invention;
Fig. 6 is a block diagram of an exemplary ultrasonic guidance auxiliary system of the present invention;
Fig. 7 is a block diagram of an exemplary ultrasonic guidance auxiliary system including an infrared light source according to the present invention; and
Fig. 8 is a block diagram of an exemplary image processing unit of the present invention.

### Detailed Description of the Embodiments

The present invention is further described below in combination with specific drawings and embodiments.

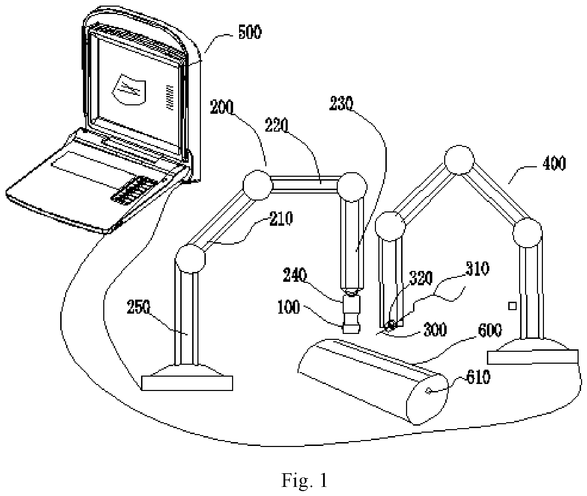

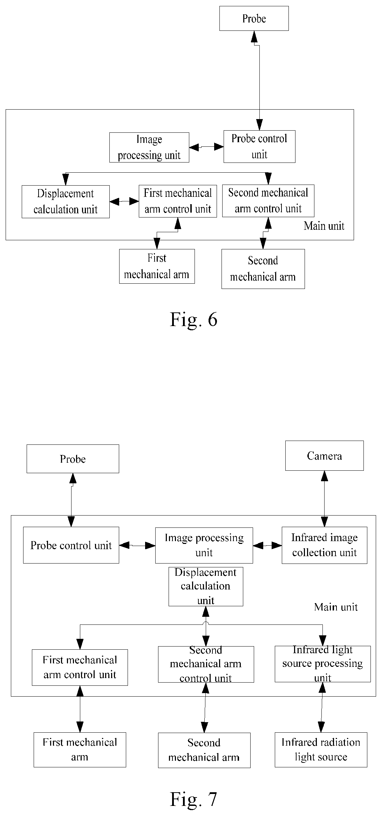

As shown in Fig. 1 and Fig. 6, an ultrasonic guidance auxiliary device for a needle includes: a probe 100, a first mechanical arm 200, a second mechanical arm 400, and a main unit 500.

The first mechanical arm 200 includes a first knuckle arm 210, a second knuckle arm 220, and a third knuckle arm 230. The first mechanical arm 200 realizes, through the first knuckle arm 210, the second knuckle arm 220 and the third knuckle arm 230, that the probe 100 rotates in three spatial dimensions of the X axis, the Y axis and the Z axis, so that the probe can quickly move to a tissue to be punctured-for-injection. When the probe 100 moves to the tissue to be punctured-for-injection, the main unit controls the probe 100 to emit and receive ultrasonic waves for the tissue to be punctured-for-injection.

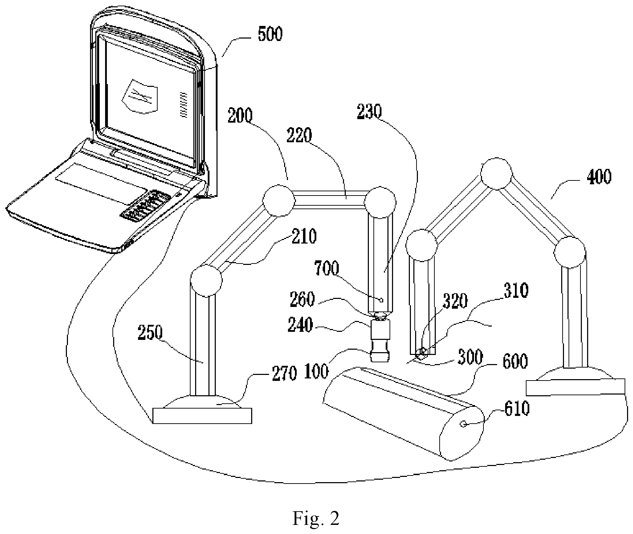

The first mechanical arm 200 and the second mechanical arm 400 are mounted on respective bases. In Fig. 2, the first mechanical arm 200 is mounted on a base 270, and the second mechanical arm 400 is mounted on the other base.

The probe 100 is used for emitting and receiving an ultrasonic signal for a human tissue to be punctured-for-injection; the first mechanical arm 200 is used for moving the probe 100 to the human tissue to be punctured-for-injection 600; a needle 300 is used for puncturing the tissue to be punctured-for-injection 600; the second mechanical arm 400 is used for fixing the needle 300, moving the needle 300 to be close to the human tissue to be punctured-for-injection 600, and then causing the needle to puncture the human tissue to be punctured-for-injection 600; and the main unit 500 is used for moving, according to controlling of the first mechanical arm 200, the probe 100 to the human tissue to be punctured-for-injection 600; and the probe 100 emits and receives the ultrasonic signal. The main unit 500 synthesizes an ultrasonic image through the ultrasonic signal transmitted by the probe 100, and the main unit 500 at least calculates a puncture distance and a puncture angle between the needle 300 and the tissue to be punctured-for-injection 600 through the ultrasonic image. The main unit controls, according to the puncture distance and the puncture angle, the second mechanical arm 400 to move the needle 300 to the corresponding puncture distance and puncture angle to perform puncturing.

A displacement calculation unit of the main unit 500 calculates, according to the ultrasonic image, a three-dimensional coordinate of a center point in the tissue to be punctured-for-injection, and the displacement calculation unit calculates, according to the three-dimensional coordinate of the center point, the puncture distance and the puncture angle between the needle and the tissue to be punctured-for-injection.

The main unit 500 acquires, according to the ultrasonic signal transmitted by the probe 100, a position of the needle 300 in the tissue to be punctured-for-injection, such as a position in a blood vessel, and controls the first mechanical arm 200 and the second mechanical arm 400 to cooperatively move to always maintain the needle 300 to a position that can be detected by the probe, and maintain the sharp end of the needle in the target region of the tissue to be punctured-for-injection, such as a target region position of the blood vessel.

For the sake of clarity, it will be understood that the phrase "the sharp end of the needle" refers to one section of the needle, which punctures a human tissue.

The second mechanical arm 400 includes a fixing device 320 of the needle 300. The fixing device can be adjusted according to different shapes of needles.

As shown in Fig. 1 and Fig. 2, the fixing device 320 of the needle 300 is arranged on the second mechanical arm, and a catheter 310 of a needle is connected with the needle. The fixing device 320 can be changed according to the shape of the needle 300.

The ultrasonic guidance auxiliary device of the present invention further includes a display. The display is a combination or one of existing displays, such as a cathode-ray tube (CRT), a liquid crystal display (LCD), a plasma screen projector, an organic light emitting diode (OLED), and a light emitting diode (LED), or other now known or later developed displays used for displaying images.

As shown in Fig. 6, in one exemplary embodiment of the present invention, an ultrasonic guidance auxiliary system for a needle includes a main unit, a first mechanical arm, a second mechanical arm, and a probe. The main unit 500 includes an image processing unit, a probe control unit, a displacement calculation unit, a first mechanical arm control unit, and a second mechanical arm control unit. The first mechanical arm control unit controls the first mechanical arm to move the probe to a human tissue to be punctured-for-injection; the probe control unit controls the probe to emit and receive the ultrasonic signal; the image processing unit synthesizes an ultrasonic image through the ultrasonic signal transmitted by the probe; the displacement calculation unit at least calculates a puncture distance and a puncture angle between the needle and the tissue to be punctured-for-injection through the ultrasonic image; the second mechanical arm control unit controls, according to the puncture distance and the puncture angle, the second mechanical arm to move the needle to the corresponding puncture distance and puncture angle to perform puncturing. The displacement calculation unit acquires, according to the ultrasonic image, a position of the needle in a blood vessel; the first mechanical arm controlled by the first mechanical arm control unit and the second mechanical arm controlled by the second mechanical arm control unit perform cooperative movement to always maintain the needle at a position that can be detected by the probe, and the sharp end of the needle is maintained in the blood vessel.

In the present embodiment, position parameters of the probe 100 are preset in the main unit 500, and the main unit 500 can automatically determine the position or quickly determine the position according to a choice of a user. For example, the user places a tissue to be punctured-for-injection of a person to be detected to a specific region, and the user makes a choice on an input device on the main unit or on an input device connected with the main unit. The main unit 500 determines an approximate position of the probe 100 according to a blood vessel or tissue selected by the user, and quickly moves the probe 100 by means of the first mechanical arm 200 to a surface of the tissue to be punctured-for-injection 600. At the same time, the main unit 500 controls the probe 100 to emit and receive ultrasonic waves, and the image processing unit (as shown in Fig. 6) in the main unit performs processing according to the obtained ultrasonic signal to obtain an ultrasonic image.

The displacement calculation unit (as shown in Fig. 6) in the main unit 500 determines parameters, such as the thickness, the depth, the diameter and the shape, of the blood vessel or tissue according to the ultrasonic image. For example, the probe 100 moves a certain displacement in a horizontal X axial direction of the tissue to be punctured-for-injection (i.e., a direction parallel to a finger), and the displacement calculation unit selects an appropriate position of the blood vessel or tissue according to the ultrasonic image of this period of distance, such as the thickest and relatively smooth position of the blood vessel. At this time, the probe 100 is moved to a certain position by the first mechanical arm 200 controlled by the main unit 500. For the convenience of understanding, this position is described as a position A.

The first mechanical arm 200 further includes a rotating articulated arm 240. The rotating articulated arm 240 may rotate the probe 100 to an appropriate angle, so that a desired probe angle can be achieved without complex displacement performed by the first knuckle arm 210, the second knuckle arm 200 and the third knuckle arm 230 of the first mechanical arm 200. The rotating articulated arm 240 is connected with the third knuckle arm 230 through a rotating joint 260.

When the first mechanical arm 200 moves the probe to the position A, the rotating articulated arm 240 rotates a certain angle, such as 30 degrees, 60 degrees, 90 degrees, 120 degrees, 150 degrees and 180 degrees, and a rotating angle and the number of rotations may also be preset. By means of the rotation of the probe 100, the displacement calculation unit obtains an appropriate rotating angle position of the probe 100 according to the ultrasonic image. The rotating articulated arm 240 rotates the probe 100 to the appropriate rotating angle position determined by the displacement calculation unit, and at this moment, the position of the probe 100 is determined, for the convenience of understanding, this position is described as a position B. At this time, the displacement calculation unit calculates parameter information, such as the depth, the diameter, the length and the shape, of the blood vessel or tissue according to the ultrasonic image. In the combination of information of a patient, such as the height, the weight, the age, the gender and a detected part, with the parameter information, such as the depth, the diameter, the length and the shape, of the blood vessel or tissue, the displacement calculation unit calculates, according to the parameter information, a displacement and a puncture angle of the needle relative to a marked position on the probe 100. The second mechanical arm 400 performs corresponding displacement and angle rotation to cause the needle 300 to reach the calculated designated position. For the convenience of understanding, this position is described as a position C. The second mechanical arm 400 causes the needle 300 to puncture the tissue to be punctured-for-injection 600 according to the puncture distance and the puncture angle which are calculated by the displacement calculation unit.

After the first mechanical arm 200 controls the probe to rotate, a multi-frame ultrasonic image of the blood vessel is obtained, the displacement calculation unit calculates a minimum sectional area of the blood vessel in the ultrasonic image through the obtained multi-frame ultrasonic image to determine whether the position of the probe at this time is orthogonal to a target blood vessel. The first mechanical arm 200 controls the probe 100 to move to the position orthogonal to the blood vessel, and the displacement calculation unit calculates the three-dimensional coordinate of the center point of the blood vessel at this time, and the displacement calculation unit converts the three-dimensional coordinate of the center point of the blood vessel into the puncture distance and the puncture angle which need to be performed by the needle controlled by the second mechanical arm 400.

When the sharp end of the needle 300 reaches a certain position, the needle is detected by the probe 100, the main unit 500 obtains an ultrasonic image of a corresponding position relation between the needle 300 and the blood vessel. As the second mechanical arm 400 continuously pushes the needle 300 to puncture the blood vessel, the displacement calculation unit calculates a distance where the first mechanical arm 200 moves along a direction opposite to a puncture direction of the needle, and controls the first mechanical arm 200 to move the probe 100 along the direction opposite to the puncture direction of the needle, so as to maintain the sharp end of the needle 300 at a preset position of the blood vessel. For example, the sharp end of the needle 300 is approximately located on a center line 630 of the blood vessel.

As shown in Fig. 2, Fig. 7, and Fig. 8: the probe 100, the first mechanical arm 200, the needle 300, the second mechanical arm 400, the main unit 500, the infrared radiation light source 700, and the camera, wherein the infrared radiation light source 700 is mounted on the third knuckle arm 230 of the first mechanical arm.

When it is ready to detect the tissue to be punctured-for-injection 600, the main unit 500 controls the infrared radiation light source 700 to irradiate infrared ray, and the camera collects a blood vessel infrared image obtained after the infrared light source irradiates the surface of the tissue to be punctured-for-injection 600; the displacement calculation unit calculates, according to the blood vessel infrared image obtained by the image processing unit, the position A at which the probe 100 needs to arrive, and the first mechanical arm control unit controls the first mechanical arm 200 to arrive at the position A by means of a moved displacement.

As shown in Fig. 7, the main unit 500 in one exemplary embodiment of the present invention includes an image processing unit, a probe control unit, a displacement calculation unit, a first mechanical arm control unit, a second mechanical arm control unit, an infrared light source processing unit and an infrared image collection unit.

The infrared light source processing unit controls the infrared radiation light source 700 to irradiate infrared ray; the infrared image collection unit controls the camera to collect a blood vessel infrared image obtained after the infrared light source irradiates the surface of the tissue to be punctured-for-injection 600; the first mechanical arm control unit controls the first mechanical arm to move the probe to a human tissue to be punctured-for-injection; the probe control unit controls the probe to emit and receive the ultrasonic signal; the image processing unit synthesizes an ultrasonic image through the ultrasonic signal transmitted by the probe; the displacement calculation unit at least calculates a puncture distance and a puncture angle between the needle and the tissue to be punctured-for-injection through the ultrasonic image; the second mechanical arm control unit controls, according to the puncture distance and the puncture angle, the second mechanical arm to move the needle to the corresponding puncture distance and puncture angle to perform puncturing. The displacement calculation unit acquires, according to the ultrasonic image, a position of the needle in a blood vessel; the first mechanical arm controlled by the first mechanical arm control unit and the second mechanical arm controlled by the second mechanical arm control unit perform cooperative movement to always maintain the needle at a position that can be detected by the probe, and the sharp end of the needle is maintained in the blood vessel.

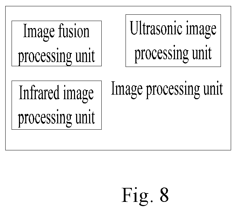

As shown in Fig. 8, the image processing unit includes: an image fusion processing unit, an ultrasonic image processing unit, and an infrared image processing unit. The image fusion processing unit fuses an ultrasonic image obtained by the ultrasonic image processing unit with an infrared image obtained by the infrared image processing unit to generate a new two-dimensional or three-dimensional or four-dimensional image, so as to more vividly display, on a display, an analog image showing the needle 300 puncturing the tissue to be punctured-for-inj ection.

The image processing unit further includes an image determination unit (not shown in the figure). The image determination unit determines the quality of an ultrasonic image by means of analyzing various pieces of ultrasonic parameter information, such as parameters of the contrast ratio, the TGC, the elastic modulus and the like, or comparing the image with an expert photo gallery in a machine. For example, if the quality of the ultrasonic image does not meet an image standard of a preset value, it is required to increase pressures on the probe 100 and the tissue to be punctured-for-injection, so that the image processing unit transmits information to the displacement calculation unit, and the first mechanical arm 200 controls the probe 100 to further move towards the tissue to be punctured-for-injection.

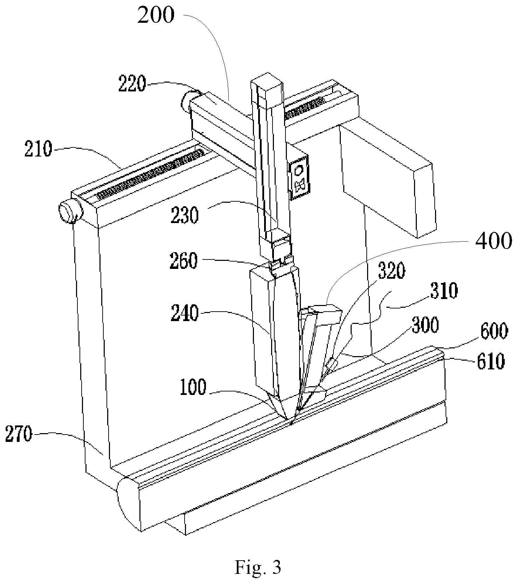

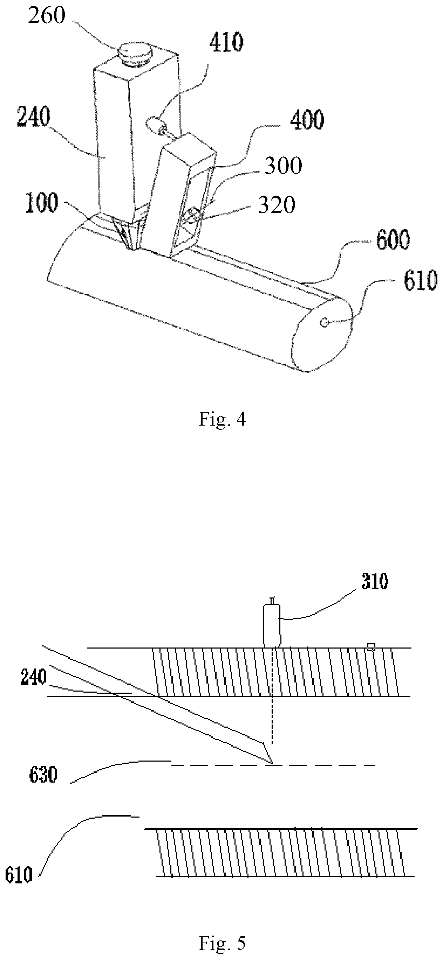

As shown in Fig. 3 and Fig. 4, in another exemplary embodiment of the present invention, the second mechanical arm 400 is mounted on the first mechanical arm 200, or the second mechanical arm 400 and the first mechanical arm 200 are jointly connected with one base 270. The second mechanical arm 400 is mounted on the first mechanical arm 200, which has small interference to the ultrasonic image and is favorable for imaging.

In Fig. 4, the second mechanical arm 400 is connected with the rotating articulated arm 240 of the first mechanical arm through one angle adjustment rod 410.

The various processing units of the main unit 500 of the present invention are processors (such as CPU and GPU). The processors are general-purpose processors, application specific integrated circuits, digital signal processors, controllers, field programmable gate arrays, digital devices, analog devices, transistors, combinations thereof or other now known or later developed for receiving analog or digital data, and outputting changed or calculated data.

In another embodiment of the present invention, a third mechanical arm is provided, and is used for controlling one or more probes, so that the one or more probes and the probe controlled by the first mechanical arm are used for ultrasonic imaging. The third mechanical arm may also be provided with an infrared radiation light source or a camera, and the like, used for emitting infrared or collecting an infrared image.

In the claims and description, the terms "first," "second," and "third," are purely used as labels, and do not attempt to require numerical values for their modifiers.

It should be noted that when an element or component is referred to as "fixing" another element or component, it can be directly on the other element or an intermediate element may also be present. When an element is considered to be "connected" to another element, it can be directly connected to the other element or an intermediate element may be present at the same time. The terms "center", "horizontal", "parallel" and similar expressions used herein are for illustrative purposes only, and are not meant to be the only embodiments.

The above is only a description of specific embodiments of the present invention, and it should be understood that those skilled in the art make substitutions and modifications through various other simple changes and equivalents without deviating from the true spirit and scope of the present utility model to achieve the objectives of the present utility model. Such modifications all fall within the scope of the appended claims.

## Claims

1. An ultrasonic guidance auxiliary device for a needle, **characterized by** comprising:
a probe, used for emitting and receiving an ultrasonic signal for a tissue to be punctured-for-inj ection;
a first mechanical arm, used for moving the probe to the tissue to be punctured-for-injection;
a second mechanical arm, used for fixing a puncture needle, moving the needle to be close to the tissue to be punctured-for-injection, and then causing the needle to puncture the tissue to be punctured-for-inj ection; and
a main unit, used for moving, according to controlling of the first mechanical arm, the probe to the tissue to be punctured-for-injection, wherein the probe emits and receives the ultrasonic signal;
the main unit synthesizes an ultrasonic image through the ultrasonic signal transmitted by the probe, and the main unit at least calculates a puncture distance and a puncture angle between the needle and the tissue to be punctured-for-injection through the ultrasonic image;
the main unit controls, according to the puncture distance and the puncture angle, the second mechanical arm to move the needle to the corresponding puncture distance and puncture angle to perform puncturing.

2. The ultrasonic guidance auxiliary device for the needle according to claim 1, **characterized in that** the main unit acquires, according to the ultrasonic signal transmitted by the probe, a position of the needle in the tissue to be punctured-for-injection, and controls the second mechanical arm to maintain the sharp end of the needle in a target region of the tissue to be punctured-for-inj ection.

3. The ultrasonic guidance auxiliary device for the needle according to claim 2, **characterized in that** the main unit controls the first mechanical arm and the second mechanical arm to cooperatively move to always maintain the sharp end of the needle to a position that can be detected by the probe, and controls the second mechanical arm to maintain the sharp end of the needle in the target region of the tissue to be punctured-for-injection.

4. The ultrasonic guidance auxiliary device for the needle according to claim 1, **characterized in that** the tissue to be punctured-for-injection comprises one or more of an artery, a vein, a nerve, and an organ.

5. The ultrasonic guidance auxiliary device for the needle according to claim 4, **characterized in that** the main unit acquires a minimum sectional area of the artery or the vein according to an ultrasound image of the artery or the vein, and the main unit controls the probe by means of the first mechanical arm to be orthogonal to the artery or the vein.

6. The ultrasonic guidance auxiliary device for the needle according to any one of claims 1 to 5, **characterized in that** the main unit calculates, according to the ultrasonic image, a three-dimensional coordinate of a center point in the tissue to be punctured-for-injection, and the main unit calculates, according to the three-dimensional coordinate of the center point, the puncture distance and the puncture angle between the needle and the tissue to be punctured-for-inj ection.

7. The ultrasonic guidance auxiliary device for the needle according to any one of claims 1 to 5, **characterized in that** the first mechanical arm comprises a rotating articulated arm, and the rotating articulated arm controls the probe to rotate to enable the main unit to obtain an ultrasonic image of the tissue to be punctured-for-injection.

8. The ultrasonic guidance auxiliary device for the needle according to any one of claims 1 to 5, **characterized in that** the ultrasonic guidance device for the needle comprises an infrared radiation light source; the infrared radiation light source is used for performing infrared radiation on the tissue to be punctured-for-injection; and the main unit further comprises an infrared image collection unit used for controlling a camera to acquire an infrared image of the tissue to be punctured-for-injection.

9. The ultrasonic guidance auxiliary device for the needle according to any one of claims 1 to 5, **characterized in that**
the second mechanical arm and the first mechanical arm are separately mounted on corresponding bases or mounted on a same base; or,
the second mechanical arm is fixedly mounted on the first mechanical arm.

10. An ultrasonic guidance auxiliary system for a needle, **characterized by** comprising:
a probe, used for emitting and receiving an ultrasonic signal for a tissue to be punctured-for-inj ection;
a first mechanical arm, used for moving the probe to the tissue to be punctured-for-injection;
a second mechanical arm, used for fixing a puncture needle, and moving the needle to the tissue to be punctured-for-injection, and then causing the needle to puncture the tissue to be punctured-for-injection; and
a main unit, wherein the main unit comprises a first mechanical arm control unit, a second mechanical arm control unit, a probe control unit, an image processing unit, and a displacement calculation unit; the first mechanical arm control unit controls the first mechanical arm to move the probe to the tissue to be punctured-for-injection, and the probe control unit controls the probe to emit and receive the ultrasonic signal;
the image processing unit synthesizes an ultrasonic image through the ultrasonic signal transmitted by the probe;
the displacement calculation unit at least calculates a puncture distance and a puncture angle between the needle and the tissue to be punctured-for-injection through the ultrasonic image;
the second mechanical arm control unit controls, according to the puncture distance and the puncture angle, the second mechanical arm to move the needle to the corresponding puncture distance and puncture angle to perform puncturing.

11. The ultrasonic guidance auxiliary system for the needle according to claim 10, **characterized in that** the displacement calculation unit acquires, according to the ultrasonic image, a position of the needle in the tissue to be punctured-for-injection, and the second mechanical arm control unit controls the second mechanical arm to maintain the sharp end of the needle in a target region of the tissue to be punctured-for-injection.

12. The ultrasonic guidance auxiliary system for the needle according to claim 10, **characterized in that**
the displacement calculation unit acquires, according to the ultrasonic image, a position of the needle in the tissue to be punctured-for-injection; the first mechanical arm controlled by the first mechanical arm control unit and the second mechanical arm controlled by the second mechanical arm control unit perform cooperative movement to always maintain the needle at a position that can be detected by the probe, and the sharp end of the needle is maintained in the target region of the tissue to be punctured-for-injection.

13. The ultrasonic guidance auxiliary system for the needle according to claim 10, **characterized by**
further comprising an infrared radiation light source, wherein the main unit further comprises an infrared light source processing unit used for controlling the infrared radiation light source to radiate infrared ray; the ultrasonic guidance auxiliary system further comprises a camera; the main unit further comprises an infrared image collection unit used for controlling the camera to acquire an infrared image of the tissue to be punctured-for-injection;
the image processing unit comprises: an image fusion processing unit, an ultrasonic image processing unit, and an infrared image processing unit; the image fusion processing unit fuses the ultrasonic image obtained by the ultrasonic image processing unit with the infrared image obtained by the infrared image processing unit to generate a new image.
